(19) Europäisches Patentamt
European Patent Office
Office européen des brevets

(11) Publication number: 0 099 165 A1

# (12) EUROPEAN PATENT APPLICATION

(21) Application number: 83300885.7

(22) Date of filing: 21.02.83

(51) Int. Cl.$^3$: C 07 D 249/08
C 07 D 233/60, C 07 D 413/06
A 01 N 43/64, A 01 N 43/50
A 01 N 43/72, A 01 N 43/76
C 07 D 303/48, C 07 D 263/14
//C07C59/115, C07C57/065,
C07C69/54, C07C69/24,
C07D263/10, C07D413/04

(30) Priority: 23.03.82 GB 8208520
08.04.82 GB 8210388
28.07.82 GB 8221757

(43) Date of publication of application:
25.01.84 Bulletin 84/4

(84) Designated Contracting States:
AT BE CH DE FR GB IT LI NL SE

(71) Applicant: IMPERIAL CHEMICAL INDUSTRIES PLC
Imperial Chemical House Millbank
London SW1P 3JF(GB)

(72) Inventor: Clough, John Martin
75 Littleworth Road
Downley High Wycombe Buckinghamshire(GB)

(72) Inventor: Worthington, Paul Anthony
4 Oakhurst Road Maidenhead Court Park
Maidenhead Berkshire(GB)

(72) Inventor: Beautement, Kevin
33 Sadlers Lane Winnersh
Wokingham Berkshire(GB)

(74) Representative: Alner, Henry Giveen Hamilton et al,
Imperial Chemical Industries PLC Legal Department:
Patents P.O. Box 6
Welwyn Garden City Herts, AL7 1HD(GB)

(54) **Triazole and imidazole compounds, process for their preparation and their use as fungicides and plant growth regulators, and intermediates for their synthesis.**

(57)

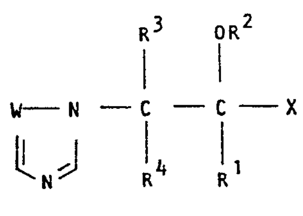

(I)

wherein $R^1$ is hydrogen, alkyl, cycloalkyl, heterocyclyl, or aryl or aralkyl both optionally substituted with halogen, nitro, alkyl, haloalkyl, alkoxy, phenyl, phenoxy, halophenyl, or haloalkoxy; $R^2$ is hydrogen, alkyl, alkenyl, alkynyl, aralkyl or acyl; $R^3$ and $R^4$, which may be the same or different, are hydrogen, alkyl, alkenyl, or optionally substituted aryl; W is N or CH; X is $-CO_2R^5$, $-CONR^6R^7$, $-CN$, $-CHO$, 4,4-dimethyl-$\Delta^2$-oxazolin-2-yl, or 4,4,6-trimethyl-5,6-dihydro-1,3-oxazin-2-yl; $R^5$, $R^6$ and $R^7$, which may be the same or different, are hydrogen, optionally substituted alkyl, optionally substituted aralkyl, alkenyl, or optionally substituted aryl; and their acid addition salts and metal complexes. The compounds have fungicidal activity and plant growth regulating activity.

EP 0 099 165 A1

JTITLE MODIFIED
see front page

# HETEROCYCLIC COMPOUNDS

This invention relates to triazole and imidazole compounds useful as fungicides, to a process for preparing them, to fungicidal compositions containing them, and to methods of using them to combat fungi, especially fungal infections in plants; and to regulate plant growth.

The invention provides a compound having the general formula (I) :

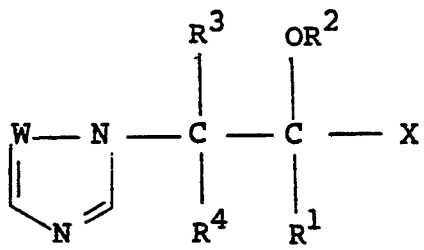

(I)

and stereoisomers thereof, wherein $R^1$ is hydrogen, alkyl, cycloalkyl or heterocyclyl, or aryl or aralkyl, both optionally substituted with halogen, nitro, alkyl, haloalkyl, alkoxy, phenyl, phenoxy, halophenyl, or haloalkoxy groups; $R^2$ is hydrogen, alkyl, alkenyl, alkynyl, aralkyl or acyl; $R^3$ and $R^4$, which may be the same or different, are hydrogen, alkyl, alkenyl, or optionally substituted aryl; W is N or CH; X is $-CO_2R^5$, $-CONR^6R^7$, -CN, -CHO, 4,4-dimethyl-$\Delta^2$-oxazolin-2-yl, or 4,4,6-trimethyl-5,6-dihydro-1,3-oxazin-2-yl in which $R^5$, $R^6$ and $R^7$, which may be the same or different, are hydrogen, optionally substituted alkyl, optionally substituted aralkyl, alkenyl, or optionally substituted aryl; and their acid addition salts and metal complexes.

The compounds have fungicidal activity and plant growth regulating activity.

In a further aspect the invention provides compounds having the formula :

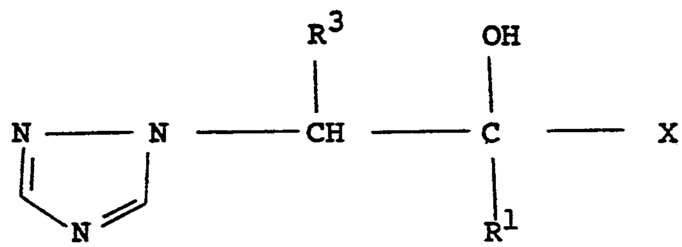

wherein $R^1$ is alkyl having from 1 to 4 carbon atoms, or phenyl optionally substituted with halogen, phenoxy, phenyl, or alkyl, haloalkyl or alkoxy having from 1 to 4 carbon atoms; $R^3$ is hydrogen, alkyl having 1 to 6 carbon atoms or phenyl; and X is $-COOR^5$ or $-CONR^6R^7$ wherein $R^5$ is alkyl or aminoalkyl having from 1 to 6 carbon atoms or phenyl and $R^6$ and $R^7$, which may be the same or different, are hydrogen, alkyl having from 1 to 6 carbon atoms, cycloalkyl or phenyl optionally substituted with halogen or alkyl or alkoxy having from 1 to 4 carbon atoms, or $R_6$ and $R_7$ together with the adjacent N-atom make up a morpholine ring.

The compounds of the invention contain at least one chiral centre. Such compounds are generally obtained in the form of isomeric mixtures. However, these and other mixtures can be separated into the individual isomers by methods known in the art.

The alkyl groups may be straight or branched chain groups having 1 to 6, eg. 1 to 4, carbon atoms; examples are methyl, ethyl, propyl (n- or iso-propyl) and butyl (n- sec-, iso- or t-butyl). Cycloalkyl groups may be cyclopropyl, cyclobutyl, cyclopentyl or cyclohexyl.

The alkenyl groups are preferably allyl, and the alkynyl groups are preferably propargyl.

Examples of suitable substituents for the aryl and

aralkyl groups, which are preferably phenyl and benzyl, are halogen (eg. fluorine, chlorine, or bromine), $C_{1-5}$ alkyl [eg. methyl, ethyl, propyl (*n*- or *iso*-propyl) and butyl (*n*-, *sec*-, *iso*- or *t*-butyl)], $C_{1-4}$ alkoxy (eg. methoxy or ethoxy), haloalkoxy (eg. trifluoromethoxy), haloalkyl (eg. trifluoromethyl), nitro, phenyl and phenoxy. The phenyl ring may be, for example, unsubstituted or substituted with 1, 2 or 3 ring substituents as defined above. The phenyl group may have a single ring substituent in the 2-, 3- or 4-position. Examples of these groups are phenyl, 2-, 3- or 4-chlorophenyl, 2,4- or 2,6-dichlorophenyl, 2-, 3- or 4-fluorophenyl, 2,4-difluorophenyl, 2-, 3- or 4-bromophenyl, 2-chloro-4-fluorophenyl, 2-chloro-6-fluorophenyl, 2-, 3- or 4-methoxyphenyl, 2-, 3- or 4-ethoxyphenyl, 2-, 3- or 4-nitrophenyl, 2-, 3- or 4-methylphenyl, 2-, 3- or 4-*t*-butylphenyl, 2-, 3- or 4-trifluoromethylphenyl, 2-, 3- or 4-phenoxyphenyl, 2-, 3- or 4-phenylphenyl (2-, 3- or 4-biphenylyl), 2-chloro-4-methylphenyl, and 2-chloro-4-methoxyphenyl.

Heterocyclyl groups may be, for example, thienyl, pyridyl, or furyl.

The acyl group is preferably acetyl or propionyl.

A particularly preferred compound according to the invention is that having the formula :

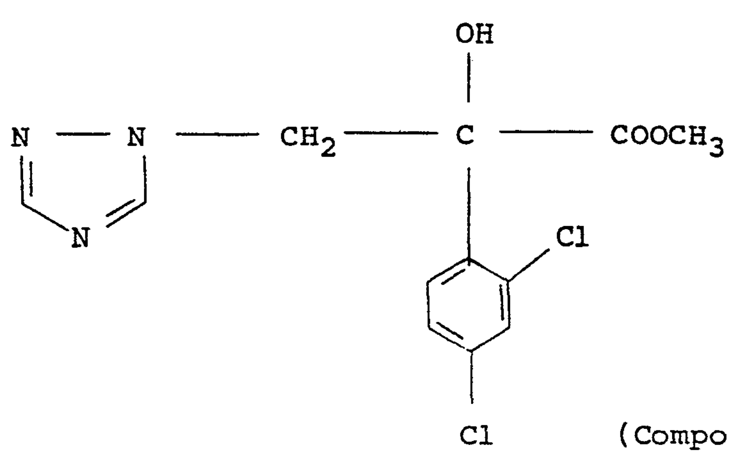

(Compound No. 7 of Table I below)

The salts can be salts with inorganic or organic acids eg. hydrochloric, nitric, sulphuric, acetic, 4-toluenesulphonic or oxalic acid.

Suitably the metal complex is one including, as the metal, copper, zinc, manganese or iron. It preferably has the general formula:

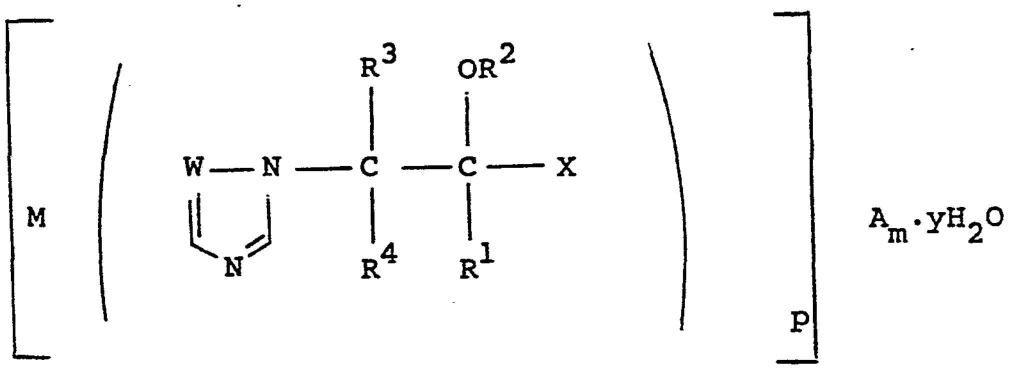

wherein W, X, $R^1$, $R^2$, $R^3$, and $R^4$, are as defined above, M is a metal, A is an anion (eg. a chloride, bromide, iodide, nitrate, sulphate or phosphate anion), p is 2 or 4, y is 0 or an integer of 1 to 12, and m is an integer consistent with valency.

Examples of the compounds of the invention are shown in Table 1. These conform to formula I.

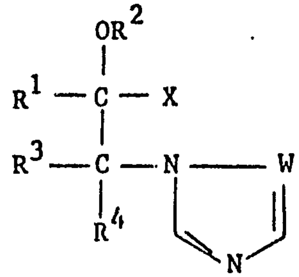

TABLE I

| COMPOUND NO | $R^1$ | $R^2$ | $R^3$ | $R^4$ | X | W | MELTING POINT (°C) |
|---|---|---|---|---|---|---|---|
| 1 | $4-Cl-C_6H_4$ | H | H | H | + | N | 104-105 |
| 2 | $C_6H_5$ | H | H | H | + | N | 113-114 |
| 3 | $2,4-di-Cl-C_6H_3$ | H | H | H | + | N | 140 |
| 4 | $2,4-di-Cl-C_6H_3$ | H | H | H | $-CO_2CH_2(CH_3)_2NH_2$ | N | 117-120 |
| 5 | $4-F-C_6H_4$ | H | H | H | + | N | 63-65 |
| 6 | $4-Cl-C_6H_4$ | H | H | H | $CONH_2$ | N | 192-193 |
| 7 | $2,4-di-Cl-C_6H_3$ | H | H | H | $CO_2CH_3$ | N | 183-184 |
| 8 | $2,4-di-Cl-C_6H_3$ | H | H | H | $CON(C_2H_5)_2$ | N | 157-158 |
| 9 | $2,4-di-Cl-C_6H_3$ | H | H | H | $CO_2(CH_2)_3CH_3$ | N | 92-93 |
| 10 | $2,4-di-Cl-C_6H_3$ | H | H | H | $CO_2CH(CH_3)(CH_2)_2CH_3$ | N | |

+ = 4,4-dimethyl-$\Delta^2$-oxazolin-2-yl

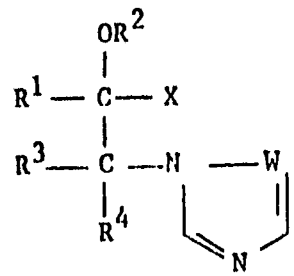

TABLE I (Cont)

| COMPOUND NO | $R^1$ | $R^2$ | $R^3$ | $R^4$ | X | W | MELTING POINT (°C) |
|---|---|---|---|---|---|---|---|
| 11 | 2,4-di-Cl-$C_6H_3$ | H | H | H | $CO_2C_2H_5$ | N | 125-126 |
| 12 | 2,4-di-Cl-$C_6H_3$ | H | H | H | $CO_2CH_2CH_2CH_3$ | N | 120-121 |
| 13 | 2,4-di-Cl-$C_6H_3$ | H | H | H | $CO_2C(CH_3)_3$ | N | 120-121 |
| 14 | 2,4-di-Cl-$C_6H_3$ | H | H | H | $CO_2CH(CH_3)_2$ | N | 148-149 |
| 15 | 2,4-di-Cl-$C_6H_3$ | H | H | H | $CO_2(C_{10}H_{21})^n$ | N | |
| 16 | 2,4-di-Cl-$C_6H_3$ | H | H | H | $CON(C_6H_5)_2$ | N | |
| 17 | 2,4-di-Cl-$C_6H_3$ | H | H | H | CON 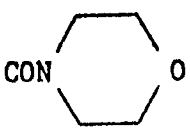 | N | 185-188 |
| 18 | 2,4-di-Cl-$C_6H_3$ | H | H | H | CONH(2-Cl-4-$CH_3$-$C_6H_3$) | N | glass |
| 19 | 2,4-di-Cl-$C_6H_3$ | H | H | H | $CO_2$(4-$C_6H_5$-$C_6H_4$) | N | |
| 20 | 2,4-di-Cl-$C_6H_3$ | H | H | H | CONH(4-$CH_3O$-$C_6H_4$) | N | glass |
| 21 | 2,4-di-Cl-$C_6H_3$ | H | H | H | CONH(4-Cl-$C_6H_4$) | N | glass |

TABLE I (Cont)

| COMPOUND NO | $R^1$ | $R^2$ | $R^3$ | $R^4$ | X | W | MELTING POINT (°C) |
|---|---|---|---|---|---|---|---|
| 22 | 2,4-di-Cl-$C_6H_3$ | H | H | H | $CONH_2$ | N | 235 decomp. |
| 23 | 2,4-di-Cl-$C_6H_3$ | H | H | H | $CO_2CH_2CH(CH_3)CH_2CH_3$ | N | Oil* |
| 24 | 2,4-di-Cl-$C_6H_3$ | H | H | H | $CO_2CH_2CH(CH_3)_2$ | N | Gum |
| 25 | 2,4-di-Cl-$C_6H_3$ | H | H | H | $CO_2C_6H_{11}$ ch | N | 111-113 |
| 26 | 2,4-di-Cl-$C_6H_3$ | H | H | H | $CO_2C_5H_9$ cp | N | 89-92 |
| 27 | 2,4-di-Cl-$C_6H_3$ | H | H | H | $COCH(CH_3)C_2H_5$ | N | 73-80* |
| 28 | 2,4-di-Cl-$C_6H_3$ | H | H | H | $CO_2CH_2CCl_3$ | N | 74-75 |
| 29 | 2,4-di-Cl-$C_6H_3$ | H | H | H | $CO_2C_6H_5$ | N | glass |
| 30 | 2,4-di-Cl-$C_6H_3$ | H | $CH_3$ | H | $CO_2CH_3$ | N | |

TABLE I (Cont)

| COMPOUND NO | $R^1$ | $R^2$ | $R^3$ | $R^4$ | X | W | MELTING POINT (°C) |
|---|---|---|---|---|---|---|---|
| 31 | 2,4-di-Cl-$C_6H_3$ | H | $C_6H_5$ | H | $CO_2CH_3$ | N | |
| 32 | $(CH_3)_3C$ | H | H | H | $CO_2CH_2CH_2CH_3$ | N | |
| 33 | 4-$CH_3O$-$C_6H_4$ | H | H | H | $CO_2CH_2CH_2CH_3$ | N | |
| 34 | 2,4-di-F-$C_6H_3$ | H | H | H | $CO_2CH_2CH_2CH_3$ | N | 62-63 |
| 35 | 2-Cl-4-$CH_3$-$C_6H_3$ | H | H | H | $CO_2CH_3$ | N | 161-162 |
| 36 | 4-$C_6H_5$-$C_6H_4$ | H | H | H | $CO_2CH_3$ | N | 130-131 |

* mixture of diastereoisomers
ch cyclohexyl
cp cyclopentyl

The compounds of the invention of general formula (I) :

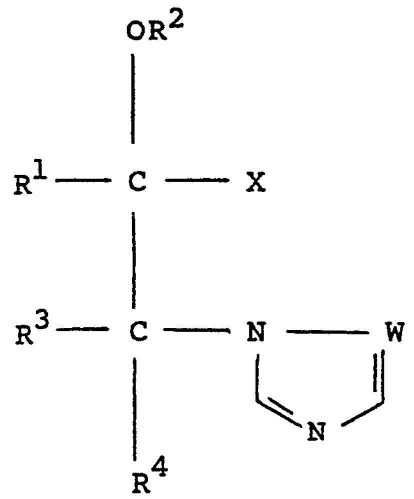

(I)

wherein X, W, $R^1$, $R^3$ and $R^4$ are as defined above and $R^2$ is hydrogen, can be prepared by treatment of epoxides of general formula (II) :

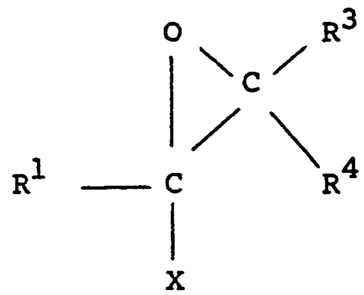

(II)

wherein X, $R^1$, $R^3$ and $R^4$ are as defined above, with 1,2,4-triazole or with imidazole, each in the presence of an acid-binding agent or in the form of one of its alkali metal salts in a convenient solvent such as dimethylformamide or acetonitrile.

Epoxides (II) wherein X is $-CO_2R^5$ or $-CONR^6R^7$, $R^5$, $R^6$ and $R^7$ being as defined above, can be prepared by treating acid halides of general formula (III) :

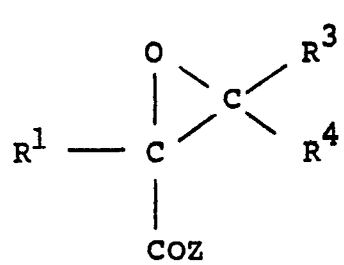

(III)

wherein $R^1$, $R^3$ and $R^4$ are as defined above and Z is a chlorine, bromine, or iodine atom, with alcohols of general formula $R^5OH$ plus a suitable base, or amines of general formula $R^6R^7NH$, respectively.

Acid halides (III) can be prepared from the corresponding acids of general formula (IV):

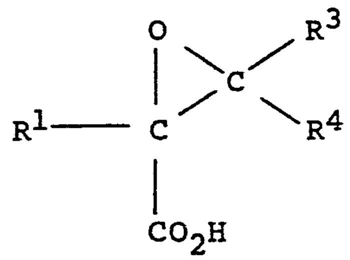

(IV)

wherein $R^1$, $R^3$ and $R^4$ are as defined above, by standard methods described in the chemical literature (see, for example, P J Beeby, Tetrahedron Letters, 1977, 3379; A L Wilds and C H Shunk, J. Amer. Chem. Soc., 1948, 2427).

Acids (IV) can be made by successive epoxidation and hydrolysis, in one flask if required, of $\alpha,\beta$-unsaturated esters of general formula (V):

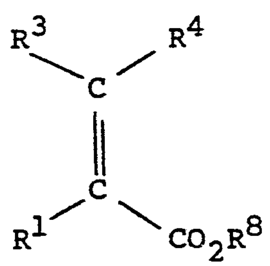

(V)

wherein $R^1$, $R^3$ and $R^4$ are as defined above and $R^8$ is an alkyl, aralkyl, cycloalkyl, alkenyl, or aryl group, using, for example, hydrogen peroxide and aqueous sodium hydroxide in a convenient solvent such as an alcohol. $\alpha,\beta$-Unsaturated esters (V) can be prepared by standard methods described in the chemical literature.

Alternatively, epoxides (II) wherein X is $-CO_2R^5$, $R^5$ being as defined above, can be prepared directly by epoxidation of unsaturated esters of general formula (V), using, for example, a peracid (such as meta-chloroperbenzoic acid) or basic hydrogen peroxide.

Alternatively, epoxides (II) can be prepared by treating halides of general formula (VI):

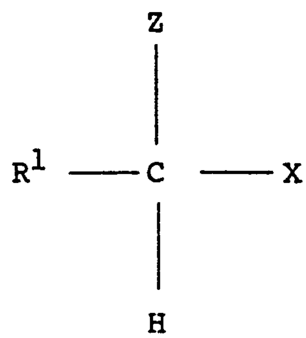

(VI)

wherein $R^1$, X and Z are as defined above, with aldehydes or ketones of general formula (VII) :

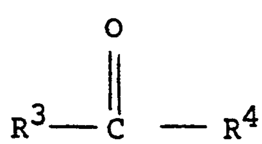

(VII)

wherein $R^3$ and $R^4$ are as defined above, under basic conditions (Darzen's reaction : see, for example, S Gladiali and F Soccolini, Synth. Commun., 1982, 12, 355; R H Hunt et al., Org. Synth., Coll. Vol. 4, 459; D M Burness, Org. Synth., Coll. Vol. 4, 649).

Halides (VI) and aldehydes or ketones (VII) can be made by standard methods described in the chemical literature.

In an alternative approach, epoxides (II) wherein X is $-CONH_2$ or -CN can be made by treatment of silylated cyanohydrins of general formula (VIII):

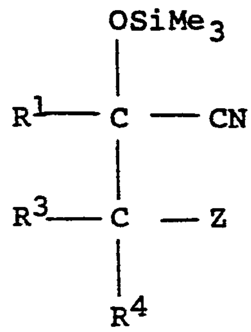

(VIII)

wherein $R^1$, $R^3$, $R^4$ and Z are as defined above, with a suitable acid. The silylated cyanohydrins (VIII) are conveniently made from α-halo ketones of general formula (IX):

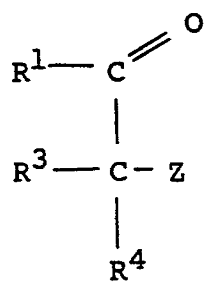

(IX)

wherein $R^1$, $R^3$, $R^4$ and Z are as defined above, by treatment with trimethylsilyl cyanide (with a catalyst, for example, zinc iodide, if required) (see, for example, P G Gassman and J J Talley, Tetrahedron Letters, 1978, 3773).

α-Halo ketones (IX) can be made by standard methods described in the chemical literature.

Epoxides (II) wherein X =

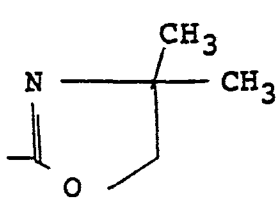

or

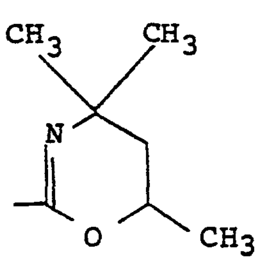

and $R^3$ = $R^4$ = H can be prepared by treating the ketones of general formula (X) :

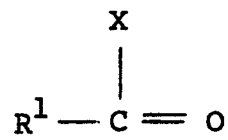 (X)

wherein X =

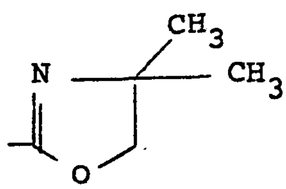 or 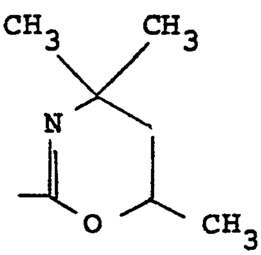

with dimethyl oxosulphonium methylide (Corey and Chaykovsky, J. Amer. Chem. Soc, 1965, 87, 1353) or dimethyl sulphonium methylide (Corey and Chaykovsky, J. Amer. Chem. Soc., 1962, 84, 3782) using methods set out in the literature.

The ketones of general formula (X) wherein X =

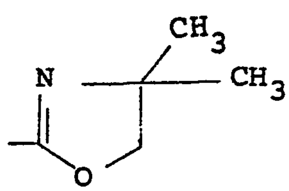 or 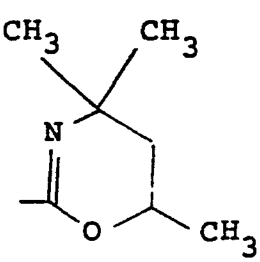

can be prepared by oxidising the compounds of general formula (XI):

$$R^1-CH_2-X \qquad (XI)$$

wherein X =

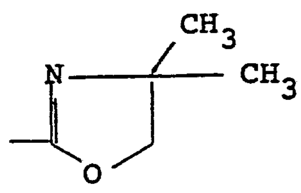

or

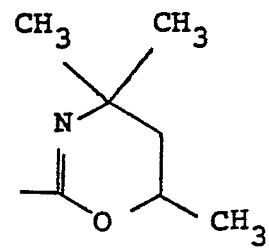

by methods set out in the literature (see, for example, J F Hansen and Su Wang, J. Org. Chem., 1976, 41, 3635-3637).

The compounds of general formula (XI) wherein X =

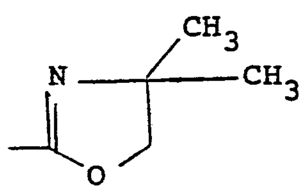

or

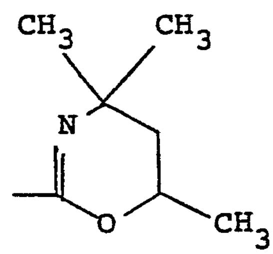

can be prepared by a variety of methods known in the literature (see, for example, A I Meyers and D L Temple Jr., J. Amer. Chem. Soc., 1970, 92, 6644 and 6646, and A I Meyers et al., J .Org. Chem., 1973, 38, 36).

Compounds of general formula (I) wherein $R^2$ is not hydrogen can be prepared from the corresponding compounds wherein $R^2$ is hydrogen by successive treatment with a base (preferably sodium hydride) and a suitable alkyl, alkenyl, alkynyl, or acyl halide (preferably chloride, bromide, or iodide) in a suitable solvent such as dimethylformamide or tetrahydrofuran.

The salts and metal complexes of the compounds of general formula (I) can be prepared from the latter by known methods. For example, the complexes can be made by reacting the uncomplexed compound with a metal salt in a suitable solvent.

The compounds, salts and metal complexes are active fungicides, particularly against the diseases:-

Piricularia oryzae on rice

Puccinia recondita, Puccinia striiformis and other rusts on wheat, Puccinia hordei, Puccinia striiformis and other rusts on barley, and rusts on other hosts eg. coffee, apples, vegetables and ornamental plants

Plasmopara viticola on vines

Erysiphe graminis (powdery mildew) on barley and wheat and other powdery mildews on various hosts such as Sphaerotheca fuliginea on cucurbits (eg. cucumber), Podosphaera leucotricha on apples and Uncinula necator on vines

Helminthosporium spp. and Rhynchosporium spp. on cereals

Cercospora arachidicola on peanuts and other Cercospora species on for example sugar beet, bananas and soya beans

Botrytis cinerea (grey mould) on tomatoes, strawberries, vines and other hosts

Phytophthora infestans (late blight) on tomatoes

Venturia inaequalis (scab) on apples

Some of the compounds have also shown a broad range of activities against fungi in vitro. They have activity against various post-harvest diseases on fruit (eg. Penicillium digatatum and italicum on oranges and Gloeosporium musarum on bananas). Further some of the compounds are active as seed dressings against: Fusarium spp., Septoria spp., Tilletia spp. (ie. bunt, a seed borne disease of wheat), Ustilago spp., Helminthosporium spp. on cereals, Rhizoctonia solani on cotton and Corticium sasakii on rice. The compounds can move acropetally in the plant tissue. Moreover, the compounds can be volatile enough

to be active in the vapour phase against fungi on the plant. They also have plant growth regulating activity.

The compounds are also useful for the treatment of candidiasis and human dermatophyte infections.

The compounds may be used as such for fungicidal purposes but are more conveniently formulated into compositions for such usage.

The invention thus further provides a fungicidal composition comprising a compound of general formula (I) as hereinbefore defined, or a salt or metal complex, thereof; and, optionally, a carrier or diluent.

The invention also provides a method of combating fungi, which comprises applying to a plant, to seed of a plant or to the locus of a plant or seed, a compound, or a salt, metal complex thereof, as hereinbefore defined, or a composition containing the same.

The invention also provides a method of regulating the growth of plants, which comprises applying to the plant, to seed of the plant, or to the locus of the plant or seed, a compound, or a salt or metal complex thereof as hereinbefore defined, or a composition containing the same.

The plant growth regulating effects of the compounds are manifested as, for example, by a stunting or dwarfing effect on the vegetative growth of woody and herbaceous mono- and di-cotyledonous plants. Such stunting or dwarfing may be useful, for example, in peanuts, cereals such as wheat and barley, oil seed rape, field beans, sunflowers, potatoes and soya bean where reduction in stem height, with or without further advantageous effects such as stem strengthening, thickening and shortening, internode shortening, increased buttress root formation and more erect stem and leaf orientation, may reduce the risk of lodging and may also permit increased amounts of fertiliser to be applied.

The stunting of woody species is useful in controlling the growth of undergrowth under power lines etc. Compounds which induce stunting or dwarfing may also be useful in modifying the stem growth of sugar cane thereby increasing the concentration of sugar in the cane at harvest; in sugar cane, the flowering and ripening may be controllable by applying the compounds. Stunting of peanuts can assist in harvesting. Growth retardation of grasses can help maintenance of grass swards. Examples of suitable grasses are Stenotaphrum secundatum (St. Augustine grass), Cynosurus cristatus, Lolium multiflorum and perenne, Agrostis tenuis, Cynodon dactylon (Bermuda grass), Dactylis glomerata, Festuca spp. (eg. Festuca rubra) and Poa spp. (eg. Poa pratense). The compounds may stunt grasses without significant phytotoxic effects and without deleteriously affecting the appearance (particularly the colour) of the grass; this makes such compounds attractive for use on ornamental lawns and on grass verges. They may also have an effect on flower head emergence in, for example,grasses. The compounds can also stunt weed species present in the grasses; examples of such weed species are sedges (eq. Cyperus spp.) and dicotyledonous weeds (eg. daisy, plantain, knotweed, speedwell, thistle, docks and ragwort). The growth of non-crop vegetation (eg. weeds or cover vegetation) can be retarded thus assisting in the maintenance of plantation and field crops. In fruit orchards, particularly orchards subject to soil erosion, the presence of grass cover is important. However excessive grass growth requires substantial maintenance. The compounds of the invention could be useful in this situation as they could restrict growth without killing the plants which would lead to soil erosion; at the same time the degree of competition for nutrients and water by the grass would be reduced and this could result in an increased yield of fruit. In some cases, one grass species

may be stunted more than another grass species; this selectivity could be useful, for example, for improving the quality of a sward by preferential suppression of the growth of undesirable species.

The dwarfing may also be useful in miniaturising ornamental, household, garden and nursery plants (eg. poinsettias, chrysanthemums, carnations, tulips and daffodils).

As indicated above, the compounds can also be used to stunt woody species. This property can be used to control hedgerows or to shape or reduce the need for pruning, of fruit trees (eg. apples, pears, cherries, peaches, vines etc). Some coniferous trees are not significantly stunted by the compounds so the compounds could be useful in controlling undesirable vegetation in conifer nurseries.

The plant growth regulating effect may (as implied above) manifest itself in an increase in crop yield; or in an ability in orchards and other crops to increase fruit set, pod set and grain set.

In the potato, vine control in the field and inhibition of sprouting in the store may be possible.

Other plant growth regulating effects caused by the compounds include alteration of leaf angle and changes in leaf morphology (both of which may permit increased light interception and utilization) and promotion of tillering in monocotyledonous plants. Improved light interception is of value in all major world crops, eg. wheat, barley, rice, maize, soya, sugarbeet, potatoes, plantation crops and orchard crops. The leaf angle effect may be useful for example in altering the leaf orientation of, for example, potato crops thereby letting more light into the crops and inducing an increase in photosynthesis and tuber weight. By increasing tillering in monocotyledonous crops (eg. rice), the number of flowering shoots per unit area may be increased thereby increasing the overall grain yield of such crops. In addition better control and modification of

hierarchical relationships is possible both in vegetative and reproductive stages of monocotyledonous and dicotyledenous plant growth, especially in cereals such as wheat, barley, rice and maize, whereby the number of flowering shoots per unit area may be increased and the size distribution of grains within the ear may be modified in such a way as to increase yield. In the treatment of rice plants, or rice crops the invention compounds can be applied, eg. as granules or a granular formulation, for example as slow release granules, to nursery boxes, paddy water and other like cultivation loci and media. In grass swards, especially amenity grass, an increase in tillering could lead to a denser sward which may result in increased resilience in wear; and to increased yields and better quality of forage grass, eg. improved digestability and palatability.

The treatment of plants with the compounds can lead to the leaves developing a darker green colour. In dicotyledonous plants such as soyabean and cotton, there may be promotion of sideshooting.

The compounds may inhibit, or at least delay, the flowering of sugar beet (and thereby may increase sugar yield) or otherwise modify the flowering patterns in many other crops. They may also reduce the size of sugar beet without reducing significantly the sugar yield thereby enabling an increase in planting density to be made. Similarly in other root crops (eg. turnip, swede, mangold, parsnip, beetroot, yam and cassava) it may be possible to increase the planting density.

The compounds could be useful in restricting the vegetative growth of cotton thereby leading to an increase in cotton yield. Crop yields may also be increased by improvement of the harvest index (ie. the harvested yield as a proportion of the total dry matter produced) by altering dry matter partitioning. This applies to all the aforementioned root, pod, cereal, tree, plantation and

orchard crops.

The compounds may be useful in rendering plants resistant to stress since the compounds can delay the emergence of plants grown from seed, shorten stem height and delay flowering; these properties could be useful in preventing frost damage in countries where there is significant snow cover in the winter since then the treated plants would remain below snow cover during the cold weather. Further the compounds may cause drought or cold resistance in certain plants.

When applied as seed treatments at low rates the compounds can have a growth stimulating effect on plants.

In carrying out the plant growth regulating method of the invention, the amount of compound to be applied to regulate the growth of plants will depend upon a number of factors, for example the particular compound selected for use, and the identity of the plant species whose growth is to be regulated. However, in general an application rate of 0.1 to 15, preferably 0.1 to 5, kg per hectare is used. With the use of biodegradable polymeric slow release granules rates of 1 to 10g per hectare are feasible; whilst electrodynamic spraying techniques may also deploy lower rates of application. However, on certain plants even application rates within these ranges may give undesired phytotoxic effects. Routine tests may be necessary to determine the best rate of application of a specific compound for any specific purpose for which it is suitable.

The compounds may be used as such for fungicidal or plant growth regulating purposes but are more conveniently formulated into compositions for such usage. The invention thus provides a fungicidal or plant growth regulating composition comprising a compound of general formula (I) as hereinbefore defined, or a salt or metal complex thereof; and, optionally, a carrier or diluent.

The invention also provides a method of combating

fungi, which comprises applying to a plant, to seed of a plant, or to the locus of the plant or seed, a compound, or salt or metal complex thereof as hereinbefore defined; or a composition containing the same.

The invention also provides a method of regulating plant growth, which comprises applying to the plant, to seed of a plant or to the locus of a plant or seed, a compound, or a salt or metal complex thereof, as hereinbefore defined, or a composition combining the same.

In the foregoing process the following compounds of Table I are especially useful:- compounds Nos. 2, 3, 7, 24, 26 and 27.

The compounds, salts, metal complexes, ethers and esters can be applied in a number of ways, for example they can be applied, formulated or unformulated, directly to the foliage of a plant, or they can be applied also to bushes and trees, to seeds or to other medium in which plants, bushes or trees are growing or are to be planted, or they can be sprayed on, dusted on or applied as a cream or paste formulation, or they can be applied as a vapour; or as slow release granules. Application can be to any part of the plant, bush or tree, for example to the foliage, stems, branches or roots, or to soil surrounding the roots, or to the seed before it is planted; or to the soil generally, to paddy water or to hydroponic culture systems. The invention compounds may also be injected into plants or trees and they may also be sprayed onto vegetation using electrodynamic spraying techniques.

The term "plant" as used herein includes seedlings, bushes and trees. Furthermore, the fungicidal method of the invention includes preventative, protectant, prophylactic and eradicant treatment.

The compounds are preferably used for agricultural and horticultural purposes in the form of a composition. The type of composition used in any instance will depend upon the particular purpose envisaged.

The compositions may be in the form of dusting powders or granules comprising the active ingredient and a solid diluent or carrier, for example fillers such as kaolin, bentonite, kieselguhr, dolomite, calcium carbonate, talc, powdered magnesia, Fuller's earth, gypsum, Hewitt's earth, diatomaceous earth and China clay. Such granules can be preformed granules suitable for application to the soil without further treatment. These granules can be made either by impregnating pellets of filler with the active ingredient or by pelleting a mixture of the active ingredient and powdered filler. Compositions for dressing seed, for example, may comprise an agent (for example a mineral oil) for assisting the adhesion of the composition to the seed; alternatively the active ingredient can be formulated for seed dressing purposes using an organic solvent (for example N-methylpyrrolidone or dimethylformamide).

The compositions may also be in the form of dispersible powders, granules or grains comprising a wetting agent to facilitate the dispersion in liquids of the powder or grains which may contain also fillers and suspending agents.

The aqueous dispersions or emulsions may be prepared by dissolving the active ingredient(s) in an organic solvent optionally containing wetting, dispersing or emulsifying agent(s) and then adding the mixture to water which may also contain wetting, dispersing or emulsifying agent(s). Suitable organic solvents are ethylene dichloride, isopropyl alcohol, propylene glycol, diacetone alcohol, toluene, kerosene, methylnaphthalene, the xylenes, trichloroethylene, furfuryl alcohol, tetrahydrofurfuryl alcohol, and glycol ethers (eg. 2-ethoxyethanol and 2-butoxyethanol).

The compositions to be used as sprays may also be in the form of aerosols wherein the formulation is held in a container under pressure in the presence of a propellant, eg. fluorotrichloromethane or dichlorodifluoromethane.

The compounds can be mixed in the dry state with a pyrotechnic mixture to form a composition suitable for generating in enclosed spaces a smoke containing the compounds.

Alternatively, the compounds may be used in a micro-encapsulated form. They may also be formulated in biodegradable polymeric formulations to obtain a slow, controlled release of the active substance.

By including suitable additives, for example additives for improving the distribution, adhesive power and resistance to rain on treated surfaces, the different compositions can be better adapted for various utilities.

The compounds can be used as mixtures with fertilisers (eg. nitrogen-, potassium- or phosphorus-containing fertilisers). Compositions comprising only granules of fertiliser incorporating, for example coated with, the compound are preferred. Such granules suitably contain up to 25% by weight of the compound. The invention therefore also provides a fertiliser composition comprising the compound of general formula (I) or a salt or metal complex thereof.

The compositions may also be in the form of liquid preparations for use as dips or sprays which are generally aqueous dispersions or emulsions containing the active ingredient in the presence of one or more surfactants eg. wetting agent(s), dispersing agent(s), emulsifying agent(s) or suspending agent(s); or which are spray formulations of the kind suitable for use in electrodynamic spraying techniques. The foregoing agents can be cationic, anionic or non-ionic agents. Suitable cationic agents are quaternary ammonium compounds, for example cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or

ammonium lignosulphonate, butylnaphthalene sulphonate, and a mixture of sodium diisopropyl- and triisopropylnaphthalene sulphonates).

Suitable non-ionic agents are the condensation products of ethylene oxide with fatty alcohols such as oleyl or cetyl alcohol, or with alkyl phenols such as octyl- or nonyl-phenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), and the concentrate is to be diluted with water before use. These concentrates often should be able to withstand storage for prolonged periods and after such storage be capable of dilution with water in order to form aqueous preparations which remain homogeneous for a sufficient time to enable them to be applied by conventional and electrodynamic spray equipment. The concentrates may conveniently contain up to 95%, suitably 10-85%, for example 25-60%, by weight of the active ingredient(s). These concentrates suitably contain organic acids (eg. alkaryl or aryl sulphonic acids such as xylenesulphonic acid or dodecyl benzenesulphonic acid) since the presence of such acids can increase the solubility of the active ingredient(s) in the polar solvents often used in the concentrates. The concentrates suitably contain also a high proportion of surfactants so that sufficiently stable emulsions in water can be obtained. After dilution to form aqueous preparations, such preparations may contain varying amounts of the active ingredient(s) depending upon the

intended purpose, but an aqueous preparation containing 0.0005% to 10%, or 0.01% to 10%, by weight of active ingredient(s) may be used.

The compositions of this invention can comprise also other compound(s) having biological activity, eg. compounds having similar or complementary fungicidal or plant growth activity or compounds having plant growth regulating, herbicidal or insecticidal activity.

The other fungicidal compound can be, for example, one which is capable of combating ear diseases of cereals (eg. wheat) such as Septoria, Gibberella and Helminthosporium spp., seed and soil borne diseases and downy and powdery mildews on grapes and powdery mildew and scab on apple etc. These mixtures of fungicides can have a broader spectrum of activity than the compound of general formula (I) alone; further the other fungicide can have a synergistic effect on the fungicidal activity of the compound of general formula (I). Examples of the other fungicidal compound are imazalil, benomyl, carbendazim, thiophanate-methyl, captafol, captan, sulphur, triforine, dodemorph, tridemorph, pyrazophos, furalaxyl, ethirimol, tecnazene, dimethirimol, bupirimate, chlorothalonil, vinclozolin, procymidone, iprodione, metalaxyl, forsetyl-aluminium, carboxin, oxycarboxin, fenarimol, nuarimol, fenfuram, methfuroxan, nitrotal-isopropyl, triadimefon, thiabendazole, etridiazole, triadimenol, biloxazol, dithianon, binapacryl, quinomethionate, guazatine, dodine, fentin acetate, fentin hydroxide, dinocap, folpet, dichlofluanid, ditalimphos, kitazin, cycloheximide, dichlobutrazol, a dithiocarbamate, a copper compound, a mercury compound, 1-(2-cyano-2-methoxyiminoacetyl)-3-ethyl urea, fenaponil, ofurace, propiconazole, etaconazole and fenpropemorph.

The compounds of general formula (I) can be mixed with soil, peat or other rooting media for the protection of plants against seed-borne, soil-borne or foliar fungal

diseases.

Suitable insecticides are Pirimor, Croneton, dimethoate, Metasystox and formothion.

The other plant growth regulating compound can be one which controls weeds or seedhead formation, improves the level or longevity of the plant growth regulating activity of the compounds of general formula (I), selectively controls the growth of the less desirable plants (eg. grasses) or causes the compound of general formula (I) to act faster or slower as a plant growth regulating agent. Some of these other agents will be herbicides.

Examples of suitable plant growth regulating compounds, which can display synergy in admixture, or use, with the invention compounds are the gibberellins (eg. $GA_3$, $GA_4$ or $GA_7$), the auxins (eg. indoleacetic acid, indolebutyric acid, naphthoxyacetic acid or naphthylacetic acid), the cytokinins (eg. kinetin, diphenylurea, benzimidazole, benzyladenine or benzylaminopurine), phenoxyacetic acids (eg. 2,4-D or MCPA), substituted benzoic acids (eg. triiodobenzoic acid), morphactins (eg. chlorfluorecol), maleic hydrazide, glyphosate, glyphosine, long chain fatty alcohols and acids, dikegulac, fluoridamid, mefluidide, substituted quaternary ammonium and phosphonium compounds (eg. chlormequat* chlorphonium or mepiquat chloride*), ethephon, carbetamide, methyl-3,6-dichloroanisate, daminozide*, asulam, abscisic acid, isopyrimol, 1-(4-chlorophenyl)-4,6-dimethyl-2-oxo-1,2-dihydropyridine-3-carboxylic acid, hydroxybenzonitriles (eg. bromoxynil), difenzoquat*, benzoylprop-ethyl 3,6-dichloropicolinic acid, and tecnazene. Synergy will be most likely to occur with those of the foregoing which are quaternary ammonium compounds and with those marked with an asterisk.

The use of the compounds of general formula (I) in conjunction with gibberellins can be useful where it is desired to reduce the plant growth regulating effects of

the compounds (eg. where they are to be used as fungicides). Where the compounds are being applied to the soil surrounding the plants or to the roots of the plant, the plant growth regulating effects of the compounds may possibly be reduced by using also certain types of phenoxybenzoic acids and their derivatives.

The following Examples illustrate the invention; the temperatures are given in degrees Centigrade (°C).

EXAMPLE 1

This Example illustrates the preparation of 2-hydroxy-2-(4-chlorophenyl)-3-(1,2,4-triazol-1-yl)propionamide (Compound No. 6 of Table I).

A mixture of 4-chlorophenacyl chloride (4.25g), trimethylsilyl cyanide (2.23g), and zinc iodide (25 mg) in dry dichloromethane (20 ml) was heated at 35-40°C under an atmosphere of nitrogen for 4 hours, adding a further portion of zinc iodide (25 mg) every hour. The solvent was removed under reduced pressure to leave a brown oil. The oil was diluted with concentrated hydrochloric acid (18 ml) then treated with gaseous hydrogen chloride while stirring at room temperature for 1 hour. The viscous reaction mixture was allowed to stand overnight, then washed with water (3 x 20 ml), and neutralised with sodium hydroxide. The mixture was extracted with ethyl acetate; the extracts were washed with water, dried over magnesium sulphate and concentrated under reduced pressure to give a sticky yellow solid (3.4g) which was triturated with ether and dried to give 2-(4-chlorophenyl)-2,3-epoxypropionamide (1.12g, 25%) as a white solid, m.p. 124-125°C, (found : C, 54.47; H, 3.98; N, 7.12%. $C_9H_8ClNO_2$ requires C, 54.68; H, 4.05; N, 7.09%).

A solution of 2-(4-chlorophenyl)-2,3-epoxypropionamide (1.07g) in dry dimethylformamide (DMF: 7 ml) was added to a

stirred solution of sodium triazole [from 1,2,4-triazole (0.51g) and sodium hydride (0.164g)] in DMF (15 ml) at room temperature under an atmosphere of nitrogen. The reaction mixture was heated at 80°C for 3 hours, allowed to cool, then poured into water and extracted with ethyl acetate. The extracts were washed with water then dried over magnesium sulphate and concentrated under reduced pressure to give a sticky white solid (1.11g) which was triturated with ethyl acetate and dried to give the title compound (0.71g, 49%) as a white solid, m.p. 192-193°C, (Found: C, 49.79; H, 4.37; N, 20,54%. $C_{11}H_{11}ClN_4O_2$ requires C, 49.54; H, 4.16; N,21.01%).

EXAMPLE 2

This Example illustrates the preparation of N,N-diethyl 2-hydroxy-2-(2,4-dichlorophenyl)-3-(1,2,4-triazol-1-yl)propionamide (Compound No. 8 of Table I).

A solution of pyruvic acid (2.2g) in dry diethyl ether (25 ml) was added to a briskly-stirred solution of 2,4-dichlorophenylmagnesium iodide [from 2,4-dichloroiodobenzene (13.65g) and magnesium (1.2g)] in ether (400 ml) under an atmosphere of nitrogen at a rate at which the ether refluxed steadily; a thick yellow precipitate formed. The mixture was stirred for 2 hours following the addition, then poured into a mixture of ice and dilute hydrochloric acid and stirred for a further 30 minutes. The ether and acidic aqueous layers were separated and the latter was extracted with fresh ether. The combined ether layers were extracted with aqeuous sodium bicarbonate and the combined extracts were washed with ether, then acidified and extracted with ether. These extracts were washed with water, dried over magnesium sulphate and concentrated to give 2-hydroxy-2-(2,4-dichlorophenyl)propionic acid (1.49g, 25%) as a yellow oil.

A mixture of 2-hydroxy-2-(2,4-dichlorophenyl)propionic

acid (0.325g) and phosphoric acid (7.5 ml) were heated at 125°C for 2 hours then allowed to cool and poured into water. The mixture was extracted with ethyl acetate and the extracts were washed with water, dried over magnesium sulphate and concentrated to give a fawn solid (0.24g), m.p. 140-143°C. Recrystallisation from 80-100° petrol gave α-(2,4-dichlorophenyl)acrylic acid (0.20g, 67%) as a colourless solid, m.p. 143-144°C

Thionyl chloride (0.28g) was added to a suspension of α-(2,4-dichlorophenyl)acrylic acid (0.34g) in dry acetonitrile (5 ml) and the mixture was heated at 80°C for 30 minutes. The volatile materials were removed under reduced pressure and the stirred residue was diluted with a solution of triethylamine (0.25g) in methanol. After 30 minutes the volatile materials were again removed under reduced pressure. Water was added to the residue and it was extracted with ether. The extracts were washed with water, treated with magnesium sulphate and charcoal, filtered and concentrated to give methyl α-(2,4-dichlorophenyl)acrylate (0.34g, 94%) as a pale yellow oil.

30% Hydrogen peroxide and a solution of sodium hydroxide (0.07g) in water (0.5 ml) were added successively to a stirred solution of methyl α-(2,4-dichlorophenyl)-acrylate (0.34g) in methanol (3 ml). After 15 minutes further sodium hydroxide (0.07g) was added. After a further 30 minutes water (10 ml) was added and the mixture was washed with ether (10 ml), acidified with dilute hydrochloric acid, and extracted with ether. The extracts were washed with water, dried over magnesium sulphate and concentrated to give 2-(2,4-dichlorophenyl)-2,3-epoxy-propionic acid (0.32g, 93%) as an oil which crystallised on standing, melting point 103-105°C (decomposed).

A solution of 2-(2,4-dichlorophenyl)-2,3-epoxy-propionic acid (1.00g) in dry ether (10 ml) was added over 10 minutes to a stirred suspension of sodium hydride

(0.104g) in ether (10 ml) under an atmosphere of nitrogen. The mixture was stirred for 1 hour at room temperature, then oxalyl chloride (0.603g) was added over 5 minutes (vigorous evolution of gas). The mixture was stirred for 45 minutes, then diethylamine (1.57g) was added over 15 minutes (thick white precipitate). The mixture was allowed to stand overnight, then poured into water and extracted with ether. The extracts were washed successively with water, aqueous sodium bicarbonate (x2), and water, then dried over magnesium sulphate and concentrated to give a yellow oil (0.83g) containing N,N-diethyl 2-(2,4-dichlorophenyl)-2,3-epoxypropionamide. A solution of the crude product in dry dimethylformamide (DMF : 5 ml) was added to a stirred solution of sodium triazole [from 1,2,4-triazole (0.21g) and sodium hydride (0.07g)] in dry DMF (10 ml) under an atmosphere of nitrogen and the mixture was heated at 80°C for 2 hours. After cooling, the mixture was poured into water and extracted with ether. The extracts were washed with water, dried over magnesium sulphate, and concentrated to give a sticky solid (0.76g). Trituration with ether and drying under reduced pressure gave the title compound [0.40g, 26% from 2-(2,4-dichlorophenyl)-2,3-epoxypropionic acid] as a white solid, m.p. 157-158°C.

## EXAMPLE 3

This Example illustrates the preparation of cyclopentyl 2-hydroxy-2-(2,4-dichlorophenyl)-3-(1,2,4-triazol-1-yl)propionate (Compound No 26 of Table I).

A solution of ethyl pyruvate (23.20g) in dry diethyl ether (100 ml) was added dropwise to a stirred solution of the Grignard reagent formed from 2,4-dichloroiodobenzene (60.00g) and magnesium (6.00g) in diethyl ether (325 ml) at a rate which maintained a steady reflux. The reaction mixture was stirred at room temperature for 1 hour, then water and dilute hydrochloric acid were added and the mixture was extracted with ether. The extracts were

washed with water, dried over magnesium sulphate and concentrated under reduced pressure to give almost pure ethyl 2-hydroxy-2-(2,4-dichlorophenyl)propionate (48.01g, 91%), $^1$H n.m.r. ($CDCl_3$) : $\delta$ 1.84 (3H,s, C$\underline{H}_3$COH).

A mixture of ethyl 2-hydroxy-2-(2,4-dichlorophenyl)-propionate (42.00g) and potassium hydroxide (16.16g) in methanol (150 ml) and water (30 ml) was stirred at room temperature for 2 days. Further water was then added to the reaction mixture and it was washed with ether, acidified with concentrated hydrochloric acid, and extracted with ether. The extracts were washed with water, dried over magnesium sulphate and concentrated to give 2-hydroxy-2-(2,4-dichlorophenyl)propionic acid (28.70g, 75%) as an orange oil.

This acid was dehydrated with phosphoric acid then converted into the corresponding acid chloride with thionyl chloride as described in Example 2. Part of the acid chloride was treated with cyclopentanol and triethylamine (as described in Example 2 for methanol and triethylamine) to give cyclopentyl $\alpha$-(2,4-dichlorophenyl)acrylate [in a yield of 52% from 2-hydroxy-2-(2,4-dichlorophenyl)-propionic acid] as a pale yellow oil, boiling point (evaporative distillation) 140°C at 0.15 torr, $^1$H n.m.r. ($CDCl_3$): $\delta$ 5.79 and 6.55 (each 1H, s, :$CH_2$).

A mixture of cyclopentyl $\alpha$-(2,4-dichlorophenyl)-acrylate (5.26g) and <u>meta</u>-chloroperbenzoic acid (6.3g) in 1,2-dichloroethane (100 ml) was heated at 100°C for 20 hours, then further portions of the peracid (0.15g) were added every hour until thin-layer chromatographic analysis showed that the unsaturated ester had gone (4 hours). The mixture was allowed to cool, diluted with dichloromethane, then washed sucessively with water (X 2), aqueous sodium bicarbonate (X 2) and water (X 2). The resulting solution was dried over magnesium sulphate and concentrated to give almost pure cyclopentyl 2-(2,4-dichlorophenyl)-2,3-epoxy-propionate (5.62g), $^1$H n.m.r. ($CDCl_3$) : $\delta$ 2.90 and 3.55

(each 1H, d, J 7Hz, epoxide ring), 5.22 (1H, br s, $CO_2CH$).

Part of this epoxide (5.50g) was added to a solution of sodium triazole [from 1,2,4-triazole (1.55g) and sodium hydride (0.48g)] in dimethylformamide (70 ml) and the mixture was heated at 160°C for 5 hours then allowed to cool, diluted with water, and extracted with ether. The extracts were washed with water, dried over magnesium sulphate, and concentrated to give an oil (4.70g). Chromatography on a column of silica gel using diethyl ether as eluant, followed by trituration, gave the title compound [2.05g, 31% from cyclopentyl α-(2,4-dichlorophenyl)acrylate] as a white solid, melting point 89-92°C, $^1H$ n.m.r. ($CDCl_3$) : δ 5.03 (2H,s, $CH_2N$), 5.29 (1H, br s, $CO_2CH$), 5.71 (1H,s, OH). (Found : C, 51.98; H, 4.47; N, 11.27%. $C_{16}H_{17}Cl_2N_3O_3$ requires C, 51.90; H, 4.63; N, 11.35%).

NOTE : Ethyl α-(2,4-dichlorophenyl)acrylate could be prepared by directly dehydrating ethyl 2-hydroxy-2-(2,4-dichlorophenyl)propionate by the following method :

Ethyl 2-hydroxy-2-(2,4-dichlorophenyl)propionate (0.50g) and phosphoric acid (5.0 ml) were mixed and heated at 125°C for 2 hours then allowed to cool. Water was added and the mixture was extracted with ether. The extracts were washed with water, dried over magnesium sulphate, and concentrated to give ethyl α-(2,4-dichlorophenyl)acrylate (0.37g, 80%) as an oil.

## EXAMPLE 4

This Example illustrates the preparation of methyl 2-hydroxy-2-(2,4-dichlorophenyl)-3-(1,2,4-triazol-1-yl)-propionate (Compound No. 7 of Table I).

A mixture of methyl (2,4-dichlorophenyl)bromoacetate (10.2g, prepared by treating methyl 2,4-dichlorophenylacetate with N-bromosuccinimide), triethylbenzylammonium chloride (0.30g), potassium carbonate (6.9g), paraformaldehyde (0.75g) and dry dimethylformamide (7.8 ml) was stirred briskly at room temperature for 60 hours. The reaction mixture was poured into water and extracted with ether. The extracts were washed with water then dried over magnesium sulphate and concentrated to give an orange oil (7.58g). Bulb-to-bulb distillation of this oil at 0.6 torr gave methyl 2-(2,4-dichlorophenyl)-2,3-epoxypropionate (3.20g, 39%) as a colourless oil (oven temperature : 155°C).

Methyl 2-(2,4-dichlorophenyl)-2,3-epoxypropionate (0.93g) was added to a solution of sodium triazole [from 1,2,4-triazole (0.29g) and sodium hydride (0.99g)] in dimethylformamide (15 ml) and the mixture was heated at 80°C for 2 hours, allowed to cool, poured into water, and extracted with ether. The extracts were washed with water, dried over magnesium sulphate, and concentrated to give a solid which was triturated with a little ether to give the title compound (0.48g, 41%) as an almost white solid, melting point 183-184°C, $^1H$ n.m.r. ($CDCl_3$) : $\delta$ 3.80 (3H, s, $CO_2CH_3$), 5.00 (2H, 2d, each J 14Hz, $CH_2N$).

EXAMPLE 5

This Example illustrates the preparation of 1-(4-chlorophenyl)-1-(4,4-dimethyl-$\Delta^2$-oxazolin-2-yl)-2-(1,2,4-triazol-1-yl)ethanol. (Compound No. 1 of Table I).

A mixture of 4-chlorophenylacetic acid (25.6g) and 2-amino-2-methyl-propan-1-ol (13.4g) and toluene (300 ml) was refluxed in a Dean and Stark apparatus for 30 hours. After cooling to room temperature the solution was washed with saturated sodium bicarbonate solution (2 x 150 ml), water (100 ml), and dried over anhydrous potassium carbonate.

BAD ORIGINAL

Removal of the solvent in vacuo gave a pale yellow oil which was distilled at reduced pressure to give 2-(4-chlorobenzyl)-4,4-dimethyl-$\Delta^2$-oxazoline (80%) as a colourless liquid, b.p. 84-85°/0.15 mm Hg.

A solution of 2-(4-chlorobenzyl)-4,4-dimethyl-$\Delta^2$-oxazoline (15.0g) in dry tetrahydrofuran (150 ml) was cooled to -72°C in a nitrogen atmosphere. n-Butyl lithium (42 ml of 1.6M solution in hexane) was added dropwise over a period of 45 minutes keeping the temperature at -70°C. Dry oxygen was bubbled into the mixture at -72°C over a period of 3 hours, and then 10% ammonium chloride solution (150 ml) was added. The mixture was extracted into diethylether; the extracts were washed with water and dried over magnesium sulphate. Removal of the ether gave a semisolid which recrystallised from 60-80 petroleum ether to give 2-(4-chlorobenzoyl)-4,4-dimethyl-$\Delta^2$-oxazoline (65%) as a pale yellow solid, m.p. 86-88°.

To a solution of dimsyl sodium [prepared by reacting sodium hydride (1.2g, 50% suspension in oil) with dry dimethyl sulphoxide (40 ml) under nitrogen at 65°C] was added dry tetrahydrofuran (40 ml) at room temperature. The solution was cooled to 0°C; trimethylsulphonium iodide (5.1g) dissolved in dimethyl sulphoxide (20 ml) was added quickly and the solution stirred for a further 1 minute. 2-(4-Chlorobenzoyl)-4,4-dimethyl-$\Delta^2$-oxazoline (4.7g) dissolved in tetrahydrofuran (20 ml) was added dropwise at 0°C over a period of 3 minutes, stirred at 0°C for 1 hour, and warmed up to room temperature for 1 hour. The mixture was poured into water and extracted with ether; the ether extracts were washed with water and dried over magnesium sulphate. Removal of the ether gave 1-(4-chlorophenyl)-1-(4,4-dimethyl-$\Delta^2$-oxazolin-2-yl) ethylene oxide (95%) as a golden yellow oil.

A solution of 1-(4-chlorophenyl)-1-(4,4-dimethyl-$\Delta^2$ oxazolin-2-yl) ethylene oxide (4.0g) in dry dimethyl formamide (10 ml) was added to a stirred solution of sodium triazole [from 1,2,4-triazole (1.65g) and sodium hydride

(0.57g)] in dimethyl formamide (50 ml) at room temperature. The reaction mixture was heated at 60° for 6 hours, allowed to cool, then poured into water and extracted with diethyl ether. The extracts were washed with water then dried over magnesium sulphate and concentrated under reduced pressure giving an orange oil which was purified by medium pressure chromatography (silica Crosfield 210 eluted with ethyl acetate) to give the title compound (40%) as a white crystalline solid m.p. 104-105°C.

EXAMPLE 6

An emulsifiable concentrate was made up by mixing the ingredients, and stirring the mixture until all the constituents were dissolved.

| | |
|---|---|
| Compound No. 7 of Table I | 10% |
| Ethylene dichloride | 40% |
| Calcium dodecylbenzenesulphate | 5% |
| "Lubrol" L | 10% |
| "Aromasol" H | 35% |

EXAMPLE 7

A composition in the form of grains readily dispersible in a liquid, eg. water, was prepared by grinding together the first three ingredients in the presence of added water and then mixing in the sodium acetate. The resultant mixture was dried and passed through a British Standard mesh sieve, size 44-100, to obtain the desired size of grains.

| | |
|---|---|
| Compound No. 7 of Table I | 50% |
| "Dispersol" T | 25% |

| | |
|---|---|
| "Lubrol" APN5 | 1.5% |
| Sodium acetate | 23.5% |

EXAMPLE 8

The ingredients were all ground together to produce a powder formulation readily dispersible in liquids.

| | |
|---|---|
| Compound No. 7 of Table I | 45% |
| "Dispersol" T | 5% |
| "Lissapol" NX | 0.5% |
| "Cellofas" B600 | 2% |
| Sodium acetate | 47.5% |

EXAMPLE 9

The active ingredient was dissolved in a solvent and the resultant liquid was sprayed on to the granules of China clay. The solvent was then allowed to evaporate to produce a granular composition.

| | |
|---|---|
| Compound No. 7 of Table I | 5% |
| China clay granules | 95% |

EXAMPLE 10

A composition suitable for use as a seed dressing was prepared by mixing the three ingredients.

| | |
|---|---|
| Compound No. 7 of Table I | 50% |
| Mineral Oil | 2% |
| China clay | 48% |

EXAMPLE 11

A dusting powder was prepared by mixing the active ingredient with talc.

| | |
|---|---|
| Compound No. 7 of Table I | 5% |
| Talc | 95% |

EXAMPLE 12

A Col formulation was prepared by ball-milling the constituents set out below and then forming an aqueous suspension of the ground mixture with water.

| | |
|---|---|
| Compound No. 7 of Table I | 40% |
| "Dispersol" T | 10% |
| "Lubrol" APN5 | 1% |
| Water | |

EXAMPLE 13

A dispersible powder formulation was made by mixing together the ingredients set out below and then grinding the mixture until all were thoroughly mixed.

| | |
|---|---|
| Compound No. 7 of Table I | 25% |
| "Aerosol" OT/B | 2% |
| "Dispersol" AC | 5% |
| China clay | 28% |
| Silica | 40% |

EXAMPLE 14

This Example illustrates the preparation of a dispersible powder formulation. The ingredients were mixed and the mixture then ground in a comminution mill.

| | |
|---|---|
| Compound No. 7 of Table I | 25% |
| "Perminal" BX | 1% |
| "Dispersol" T | 5% |
| Polyvinylpyrrolidone | 10% |
| Silica | 25% |
| China clay | 34% |

EXAMPLE 15

The ingredients set out below were formulated into a dispersible powder by mixing then grinding the ingredients.

| | |
|---|---|
| Compound No. 7 of Table I | 25% |
| "Aerosol" OT/B | 2% |
| "Dispersol" A | 5% |
| China clay | 68% |

In Examples 6 to 15 the proportions of the ingredients given are by weight.

The remaining compounds of Table I were formulated in the same way as described in Examples 6 to 15.

There now follows an explanation of the compositions or substances represented by the various Trade Marks and Trade Names mentioned above.

| | |
|---|---|
| LUBROL L : | a condensate of nonyl phenol (1 mole) with ethylene oxide (13 moles) |
| AROMASOL H : | a solvent mixture of alkylbenzenes |
| DISPERSOL T & AC : | a mixture of sodium sulphate and a condensate of formaldehyde with sodium |

| | |
|---|---|
| | naphthalene sulphonate |
| LUBROL APN5 : | a condensate of nonyl phenol (1 mole) with naphthalene oxide (5.5 moles) |
| CELLOFAS B600 : | a sodium carboxymethyl cellulose thickener |
| LISSAPOL NX : | a condensate of nonyl phenol (1 mole) with ethylene oxide (8 moles) |
| AEROSOL OT/B : | dioctyl sodium sulphosuccinate |
| PERMINAL BX : | a sodium alkyl naphthalene sulphonate |

EXAMPLE 16

The compounds were tested against a variety of foliar fungal diseases of plants. The technique employed was as follows.

The plants were grown in John Innes Potting Compost (No 1 or 2) in 4 cm diameter minipots. A layer of fine sand was placed at the bottom of the pots containing the dicotyledonous plants to facilitate uptake of test compound by the roots. The test compounds were formulated either by bead milling with aqueous Dispersol T or as a solution in acetone or acetone/ethanol which was diluted to the required concentration immediately before use. For the foliage diseases, suspensions (100 ppm active ingredient) were sprayed on to the soil. Exceptions to this were the tests on Botrytis cinerea, Plasmopara viticola and Venturia inaequalis. The sprays were applied

to maximum retention and the root drenches to a final concentration equivalent to approximately 40 ppm a.i./dry soil. Tween 20, to give a final concentration of 0.05%, was added when the sprays were applied to cereals.

For most of the tests the compound was applied to the soil (roots) and to the foliage (by spraying) one or two days before the plant was inoculated with the diseases. An exception was the test on Erysiphe graminis in which the plants were inoculated 24 hours before treatment. After inoculation, the plants were put into an appropriate environment to allow infection to take place and then incubated until the disease was ready for assessment. The period between inoculation and assessment varied from four to fourteen days according to the disease and environment.

The disease control was recorded by the following grading:-

4 = no disease
3 = trace - 5% of disease on untreated plants
2 = 6-25% of disease on untreated plants
1 = 26-59% of disease on untreated plants
0 = 60-100% of disease on untreated plants

The results are shown in Table II.

TABLE II

| COMPOUND NUMBER | PUCCINIA RECONDITA (WHEAT) | ERYSIPHE GRAMINIS (BARLEY) | PIRICULARIA ORYZAE (RICE) | PLASMOPARA VITICOLA (VINE) | PHYTOPHTHORA INFESTANS (TOMATO) | BOTRYTIS CINEREA (GRAPE) | CERCOSPORA ARACHIDICOLA (PEANUT) | VENTURIA INAEQUALIS (APPLE) |
|---|---|---|---|---|---|---|---|---|
| 1 | 4 | 4 | - | 3 | - | 0 | 3 | 4 |
| 2 | 4 | 4 | - | 0 | - | 0 | 0 | 0 |
| 3 | 4 | 4 | - | 0 | - | 0 | 4 | 4 |
| 4 | 4 | 4 | - | 0 | - | 0 | 3 | 4 |
| 5 | 4 | 4 | 0 | 0 | - | 0 | 2 | 0 |
| 6 | 4 | 4 | - | 0 | - | 0 | 3 | 4 |
| 7 | 4 | 4 | - | 0 | - | 0 | 4 | 4 |
| 8 | 4 | 4 | 2 | 0 | - | 0 | 4 | 4 |
| 9 | 4 | 4 | 3 | 1 | - | 0 | 4 | - |
| 11 | 4 | 4 | 4 | 0 | - | 0 | 4 | 4 |
| 12 | 4 | 4 | 4 | 2 | - | 0 | 4 | - |
| 14 | 4 | 4 | 4 | 1 | - | 0 | 3 | - |

TABLE II

| COMPOUND NUMBER | PUCCINIA RECONDITA (WHEAT) | ERYSIPHE GRAMINIS (BARLEY) | PIRICULARIA ORYZAE (RICE) | PLASMOPARA VITICOLA (VINE) | PHYTOPHTHORA INFESTANS (TOMATO) | BOTRYTIS CINEREA (GRAPE) | CERCOSPORA ARACHIDICOLA (PEANUT) | VENTURIA INAEQUALIS (APPLE) |
|---|---|---|---|---|---|---|---|---|
| 17 | 4 | 4 | 1 | 0 | - | 0 | 4 | 4 |
| 18 | 4 | 4 | 3 | 1 | - | 0 | 4 | - |
| 20 | 4 | 4 | 3 | 0 | - | 0 | 4 | - |
| 21 | 4 | 4 | 1 | 0 | - | 0 | 4 | - |
| 22 | 4 | 4 | 0 | 2 | - | 0 | 4 | 4 |
| 23 | 4 | 4 | 3 | 1 | - | 0 | 4 | - |
| 24 | 4 | 4 | 3 | 3 | - | - | 4 | 4 |
| 25 | 3 | 4 | 2 | 0 | - | - | 3 | 4 |
| 26 | 4 | 4 | 4 | 0 | - | - | 4 | 4 |
| 27 | 4 | 4 | 4 | 1 | - | - | 4 | 4 |
| 28 | 4 | 4 | 3 | 2 | - | - | 4 | 4 |

EXAMPLE 17

This Example illustrates the plant growth regulating properties of the compounds. The compounds were applied as an overall spray of an emulsifiable concentrate diluted to give the concentration shown in Table III. The plants were grown in 3" pots in peat compost and sprayed at the 2 leaf stage. Plant growth regulating effects were assessed 18-19 days after application of the compounds. Retardation of growth was scored on a 0-3 scale where :

1 = 0-30% retardation
2 = 31-75% retardation
3 = 75% retardation

Additional plant growth regulating properties are indicated as follows :

G = darker green leaf colour
A = apical effect
T = tillering/sideshooting effect

The results are shown in Table III. If no figure is shown the compound was substantially inactive as a stunting agent.

TABLE III

| COMPOUND | DAT | RATE (ppm) | AT | CC | DA | LT | SB | TO | SY | CT | MZ | WW | BR |
|---|---|---|---|---|---|---|---|---|---|---|---|---|---|
| 2 | 19 | 4000 | | | | 1G | 3G | 1 | 1 | G | | 2 | 2T |
| 3 | 19 | 4000 | 2 | 2G | 2G | 3A | 3GA | 3G | 2G | 3GAT | 2 | 2 | 1 |
| 7 | 19 | 4000 | 1 | 1 | 1 | 1 | 3G | 2G | 1 | 1 | 1 | 2GT | 2G |
| 24 | 18 | 4000 | 2G | 2G | 2G | 3GA | 3GA | 3GAT | 3GAT | 1GAT | 3G | | T |
| 26 | 19 | 4000 | 3G | 2 | 1G | 2GA | 2G | 3G | 2GA | 3GA | 1 | 1 | 1 |
| 27 | 18 | 4000 | 3G | 3G | 2G | 2GA | 2G | 3G | 2GA | 3GA | 2 | 1 | 1 |

Key to test species in Table III

| | |
|---|---|
| AT | *Agrostis tenuis* |
| CC | *Cynosurus cristatus* |
| DA | *Dactylis glomerata* |
| LT | *Lactuca sativa* |
| SB | *Beta vulgaris* |
| TO | *Lycopersicum esculentum* |
| SY | *Glycine max* |
| CT | *Gossypium hirsutum* |
| MZ | *Zea mays* |
| WW | *Triticum aestivum* |
| BR | *Hordeum vulgare* |

HGHA/jlw
SPEC 338
10 Feb 83

1. Compounds having the general formula (I) :

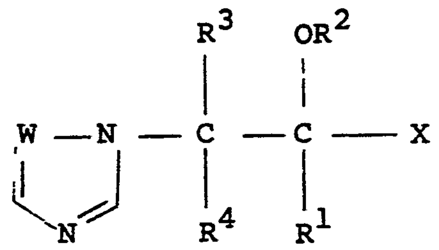

(I)

and stereoisomers thereof, wherein $R^1$ is hydrogen, alkyl, cycloalkyl or heterocyclyl, or aryl or aralkyl both optionally substituted with halogen, nitro, alkyl, haloalkyl, alkoxy, phenyl, phenoxy, halophenyl, or haloalkoxy groups; $R^2$ is hydrogen, alkyl, alkenyl, alkynyl, aralkyl or acyl; $R^3$ and $R^4$, which may be the same or different, are hydrogen, alkyl, alkenyl, or optionally substituted aryl; W is N or CH; X is $-CO_2R^5$, $-CONR^6R^7$, -CN, -CHO, 4,4-dimethyl-$\Delta^2$-oxazolin-2-yl, or 4,4,6-trimethyl-5,6-dihydro-1,3-oxazin-2-yl in which $R^5$, $R^6$ and $R^7$, which may be the same or different, are hydrogen, optionally substituted alkyl, optionally substituted aralkyl, alkenyl, or optionally substituted aryl; and their acid addition salts and metal complexes.

2. Compounds having the formula :

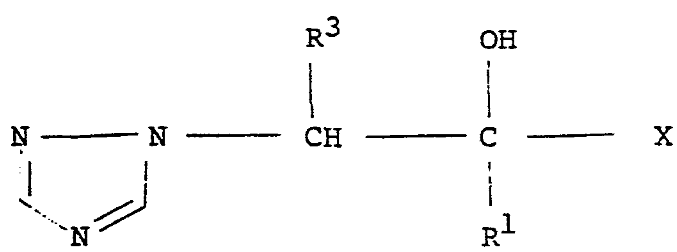

wherein $R^1$ is alkyl having from 1 to 4 carbon atoms or phenyl optionally substituted with halogen, phenoxy, phenyl, or alkyl, haloalkyl or alkoxy having from 1 to 4 carbon atoms; $R^3$ is hydrogen, alkyl having 1 to 6 carbon atoms or phenyl; and X is $-COOR^5$ or $-CONR^6R^7$ wherein $R^5$ is alkyl or aminoalkyl having from 1 to 6 carbon atoms or phenyl and $R^6$ and $R^7$, which may be the same or different, are hydrogen, alkyl having from 1 to 6 carbon atoms, cycloalkyl or phenyl opti -a y substituted with halogen or alkyl or alkoxy having from 1 to 4 carbon atoms, or $R_6$ and $R_7$ together with the adjacent N-atom make up a morpholine ring.

3. Compounds as claimed in claim 1 or claim 2 wherein, when any of $R^1$, $R^2$, $R^3$, $R^4$, $R^5$, $R^6$ and $R^7$ is alkyl, it is a straight or branched chain alkyl group having 1 to 6 carbon atoms, especially a methyl, ethyl, propyl (n- or iso-propyl) or butyl (n-sec-, iso- or t-butyl) group.

4. Compounds as claimed in any of the preceding claims wherein, when any of $R^1$, $R^2$, $R^3$, $R^4$, $R^5$, $R^6$, or $R^7$ is alkenyl or alkynyl it is allyl or propargyl respectively; and when any of $R^1$, $R^2$, $R^3$, $R^4$, $R^5$, $R^6$ or $R^7$ is cycloalkyl it is cyclopropyl, cyclobutyl, cyclopentyl or cyclohexyl.

5. Compounds as claimed in any of the preceding claims wherein, when any of $R^1$, $R^2$, $R^3$, $R^4$, $R^5$, $R^6$ and $R^7$, as appropriate, is optionally substituted aryl or aralkyl it is phenyl or benzyl both optionally substituted with halogen (eg. fluorine, chlorine or bromine), $C_{1-5}$ alkyl [eg. methyl, ethyl, propyl (n- or iso-propyl) or butyl (n-, sec-, iso- or t-butyl)], $C_{1-4}$ alkoxy (eg. methoxy or ethoxy), haloalkoxy (eg. trifluoromethoxy), haloalkyl (eg.

trifluoromethyl), nitro, phenyl or phenoxy.

6. Compounds as claimed in claim 4 wherein any of $R^1$ to $R^7$, as appropriate, is phenyl, 2-, 3- or 4-chlorophenyl, 2,4- or 2,6-dichlorophenyl, 2-, 3- or 4-fluorophenyl, 2,4-difluorophenyl, 2-, 3- or 4-bromophenyl, 2-chloro-4-fluorophenyl, 2-chloro-6-fluorophenyl, 2-, 3- or 4-methoxyphenyl, 2-, 3- or 4-ethoxyphenyl, 2-, 3- or 4-nitrophenyl, 2-, 3- or 4-methylphenyl, 2-, 3- or 4-t-butylphenyl, 2-, 3- or 4-trifluoromethylphenyl, 2-, 3- or 4-phenoxyphenyl, 2-, 3- or 4-phenylphenyl (2-, 3- or 4-biphenylyl), 2-chloro-4-methylphenyl, or 2-chloro-4-methoxyphenyl; or benzyl or a corresponding substituted benzyl group.

7. The compound of structural formula :

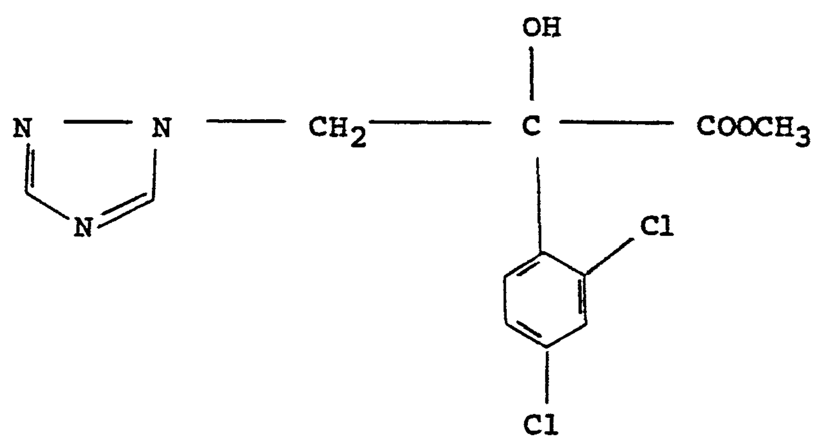

8. A process for preparing the compounds of the invention of general formula (I) :

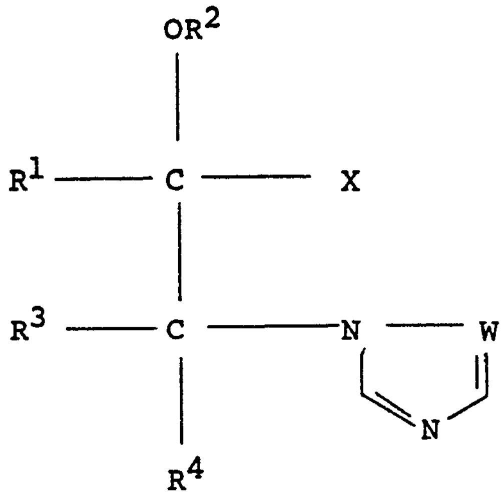

(I)

wherein X, W, $R^1$, $R^3$ and $R^4$ are as defined above and $R^2$ is hydrogen, which comprises reacting epoxides of general formula (II) :

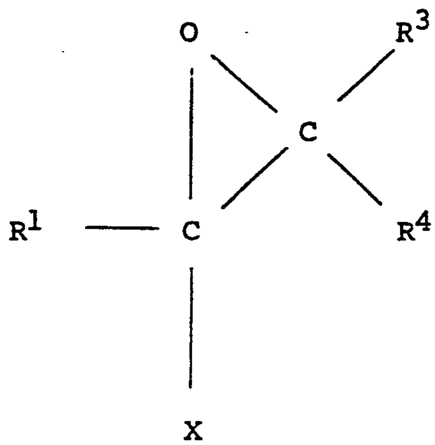

(II)

wherein X, $R^1$, $R^3$ and $R^4$ are as defined above, with 1,2,4-triazole or with imidazole, each in the presence of an acid-binding agent or in the form of one of its alkali metal salts in a convenient solvent such as dimethylformamide or acetonitrile.

9. A process according to claim 8 wherein

(a) epoxides (II) wherein X is $-CO_2R^5$ or $-CONR^6R^7$ and $R^5$, $R^6$ and $R^7$ are as defined above are prepared by treating acid halides of general formula (III) :

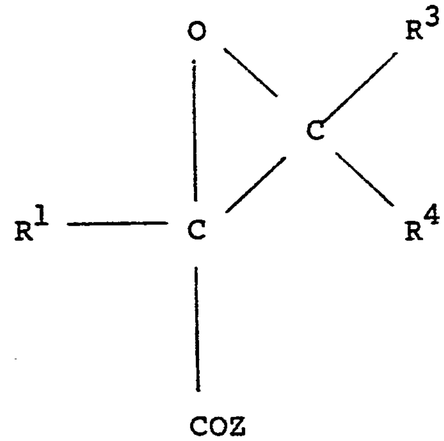

(III)

wherein $R^1$, $R^3$ and $R^4$ are as defined above and Z is a chlorine, bromine or iodine atom, with alcohols of general formula $R^5OH$ plus a suitable base, or with amines of general formula $R^6R^7NH$, respectively; the acid halides of formula (III) being themselves prepared by halogenation of the corresponding acids of general formula (IV) :

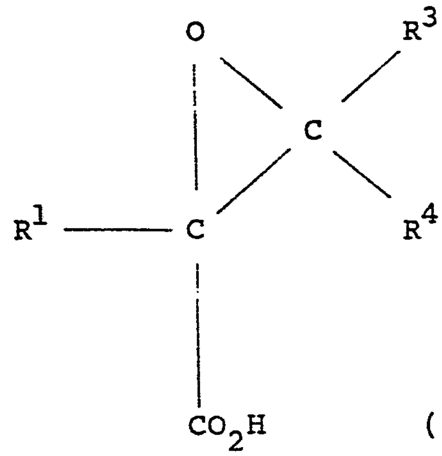

(IV)

(b) epoxides (II) wherein X is $-CO_2R^5$ and $R^5$ is as defined above, are prepared by epoxidation of unsaturated esters of general formula (V) :

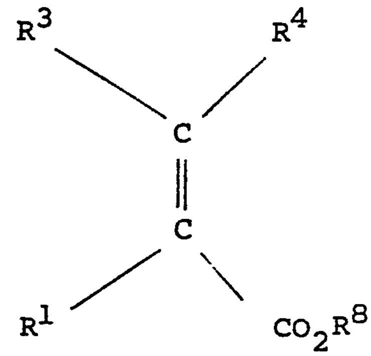

wherein $R^1$, $R^3$, $R^4$ and $R^8$ are as defined above, using, for example, a peracid (such as meta-chloroperbenzoic acid) or basic hydrogen peroxide;

(c) epoxides (II) are prepared by treating halides of general formula (VI) :

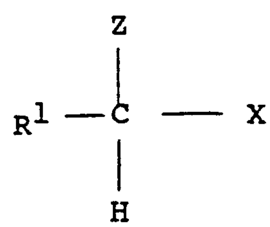

(VI)

wherein $R^1$, X and Z are as defined above, with aldehydes or ketones of general formula (VII) :

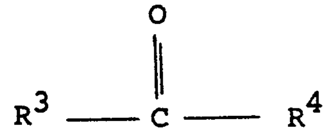

(VII)

wherein $R^3$ and $R^4$ are as defined above, under basic conditions;

(d) epoxides (II) wherein X is $-CONH_2$ or -CN are made by treatment of silylated cyanohydrins of general formula (VIII) :

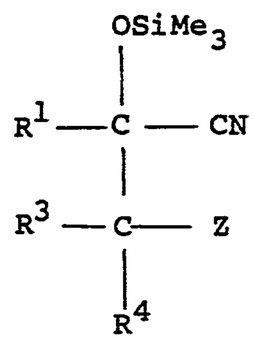

(VIII)

wherein $R^1$, $R^3$, $R^4$ and Z are as defined above, with a suitable acid, the silylated cyanohydrins (VIII) themselves being conveniently made by treatment of α-halo ketones of general formula (IX) :

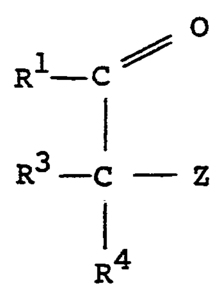

(IX)

wherein $R^1$, $R^3$, $R^4$ and Z are as defined above, with trimethylsilyl cyanide and, if necessary, a catalyst, for example zinc iodide; or

(e) epoxides (II) wherein X is :

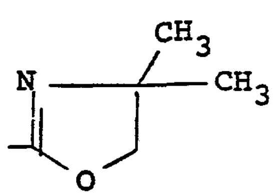

or

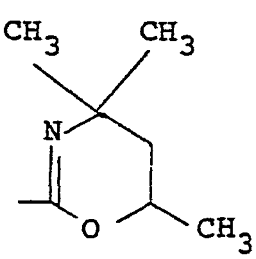

and $R^3 = R^4 = H$ are prepared by treating the ketones of general formula (X) :

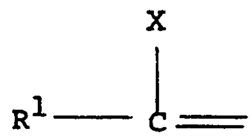

(X)

wherein X =

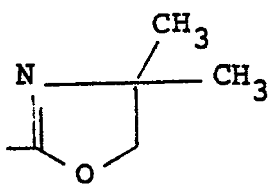

or

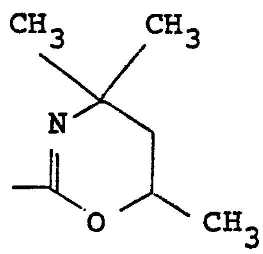

with dimethyl oxosulphonium methylide or dimethyl sulphonium methylide using methods set out in the literature.

10. A process for preparing the compounds of the invention of general formula (I) :

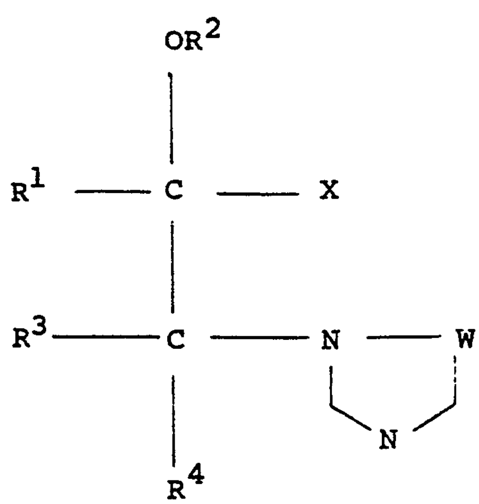

wherein X, W, $R^1$, $R^3$ and $R^4$ are as defined in claim 1 and wherein $R^2$ is other than hydrogen which comprises subjecting compounds wherein $R^2$ is hydrogen to successive treatment with a base (preferably sodium hydride) and a suitable alkyl, alkenyl, alkynyl, or acyl halide (preferably chloride, bromide, or iodide) in a suitable solvent such as dimethyl formamide or tetrahydrofuran.

11. A fungicidal, or plant growth regulating composition comprising a compound of general formula (I) as defined in any of claims 1 to 7 or a salt or complex thereof, and a carrier or diluent.

12. A method of regulating the growth of plants, which comprises applying to the plant, to seed of the plant, or to the locus of the plant or seed, a compound, or a salt or complex thereof, as defined in any of claims 1 to 7 or a composition as defined in claim 11.

13. A method of combating fungal diseases in a plant, which method comprises applying to the plant, to seed of the plant or to the locus of the plant or seed, a compound, or a salt or complex thereof, as defined in any of claims 1 to 7 or a composition as defined in claim 11.

14. The compounds of formulae (II), (III), (IV), (VIII), and (X) as defined.

0099165

European Patent Office

# EUROPEAN SEARCH REPORT

Application number

EP 83 30 0885

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| P,X | EP-A-0 071 568 (CIBA GEIGY)<br>* Claims; pages 67-74; compound 6.2, tabel 6, page 53; compound 1.1, tabel 1, page 31 *<br>--- | 1-7,10-13 | C 07 D 249/08<br>C 07 D 233/60<br>C 07 D 413/06<br>A 01 N 43/64<br>A 01 N 43/50<br>A 01 N 43/72<br>A 01 N 43/76<br>C 07 D 303/48<br>C 07 D 263/14 //<br>C 07 C 59/115<br>C 07 C 57/065<br>C 07 C 69/54<br>C 07 C 69/24<br>C 07 D 263/10<br>C 07 D 413/04 |
| Y | DE-A-2 242 454 (BAYER)<br>* Claims *<br>--- | 1,3-5 | |
| Y | FR-A-2 246 548 (BAYER)<br>* Claims *<br>----- | 1 | |
| | | | TECHNICAL FIELDS SEARCHED (Int. Cl 3) |
| | | | C 07 D 249/00<br>C 07 D 233/00<br>C 07 D 413/00 |

The present search report has been drawn up for all claims

| Place of search | Date of completion of the search | Examiner |
|---|---|---|
| THE HAGUE | 11-07-1983 | CREMERS K. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document